# EUROPEAN PATENT SPECIFICATION

(11) **EP 3 886 959 B1**
(45) Date of publication and mention of the grant of the patent: **13.03.2024**
(21) Application number: 19816899.9
(22) Date of filing: 26.11.2019
(51) Int. Cl.: A61M 25/00, F16L 11/08

(54) **ENDOSCOPIC SYSTEM FOR ENERGY DELIVERY**
ENDOSKOPISCHES SYSTEM ZUR ENERGIEBEREITSTELLUNG
SYSTÈME ENDOSCOPIQUE D'ADMINISTRATION D'ÉNERGIE

(30) Priority: 27.11.2018 US 201862771825 P
(43) Date of publication of application: 06.10.2021
(73) Proprietor: Neuwave Medical, Inc., Madison, WI 53704 (US)
(72) Inventor: THOM, Mark, Madison, Wisconsin 53704 (US); THIEL, Matthew, Madison, Wisconsin 53704 (US); MINGIONE, Louie, Madison, Wisconsin 53704 (US)
(74) Representative: Murgitroyd & Company
(86) International application number: PCT/IB2019/060186
(87) International publication number: WO 2020/109999

(56) References cited:
- EP-A1- 0 277 366
- WO-A1-2017/024289
- US-A- 5 234 416
- US-A- 5 782 760
- US-A1- 2003 028 173
- US-A1- 2004 167 496
- US-A1- 2005 137 519
- US-A1- 2009 165 881
- US-A1- 2010 268 173
- US-A1- 2012 277 729
- US-A1- 2013 046 298
- US-B1- 6 213 995

## Description

### FIELD OF INVENTION

The present invention relates to flexible sheathes configured to withstand high amounts of temperature during endoscopic energy delivery procedures, and related systems. In particular, the present invention provides a sheath having a flexible elongate tubular body designed with a temperature resistant polymer and/or a temperature resistant braided material. Such sheathes may be configured for use in any kind of endoscopic energy delivery procedure (e.g., tissue ablation, resection, cautery, vascular thrombosis, treatment of cardiac arrhythmias and dysrhythmias, electrosurgery, tissue harvest, etc.).

### BACKGROUND

Flexible sheaths are used in therapeutic endoscopy procedures (e.g., endoscopic ablation procedures) to provide more precise access to a target location than an endoscope can provide. Current or typical flexible sheaths (e.g., having a polymer material (e.g., polyether block amide (Pebax)) and a metal braiding (e.g., a stainless steel metal braiding)) must be retracted in such endoscopic ablation procedures as such sheaths are incapable of handling high temperatures associated with ablation energy (e.g., microwave ablation energy). Indeed, such sheaths must be retracted prior to delivery of ablation energy to prevent damage to the sheath from the energy delivery (e.g., microwave field resulting from microwave energy delivery). Such necessary flexible sheath retraction, however, often compromises the endoscopic ablation procedure through, for example, misadjusting the target location and/or misadjusting the positioning of the energy delivery device.

US 2009/165881 A1 describes a method of manufacturing a catheter assembly that generally includes providing a catheter shaft having an outer layer and an inner reinforcing layer; removing at least a portion of the outer layer from a length of the distal end of the catheter shaft in order to expose a distal segment thereof; providing an inner jacket segment; axially engaging the inner jacket segment with an interior surface of the distal segment of the catheter shaft; providing an outer jacket segment around at least the exposed exterior region of the distal segment of the catheter shaft; and bonding the distal segment of the catheter shaft to the inner jacket segment and the outer jacket segment.

WO 2017/024289 A1 describes catheters, sheaths, or other tubular devices that include a proximal end, a distal end sized for introduction into a patient's body, and a steerable distal portion. The tubular device includes a primary lumen extending between the proximal and distal ends; an auxiliary lumen adjacent the primary lumen; and one or more reinforcement members including windings extending helically along at least the distal portion, at least some of the windings passing between the primary and steering element lumens and at least some of the windings surrounding both the primary and steering element lumens. A steering element is slidably disposed within the auxiliary lumen.

US 2003/028173 A1 describes an intravascular catheter including a reinforcement layer having a plurality of interwoven metal wire members and polymer members. The polymer members are thermally processed to permeate the metal wire members to form a polymeric layer having an orientation which is interwoven with the metal wire members.

US 5 234 416 A describes an intravascular catheter such as a guiding catheter of composite construction having a nontraumatic distal tip comprising a proximal elastomeric tubular element and a distal elastomeric tubular element formed of softer material that the proximal section. The proximal tubular section of the distal tip preferably has a radiopaque material incorporated therein to enable the distal tip to be fluoroscopically observable when in place within a patient. The shaft of the catheter, which exhibits excellent torquability and pushability, is formed with a very thin wall. The catheter shaft includes an inner tubular member of braided polymeric fibrous strands impregnated with a thermoset polyurethane and having an outer jacket or coating of thermoplastic polyurethane secured to the braided tubular member. A lubricous liner is included within the braided tubular member.

US 2004/167496 A1 describes medical catheters adapted for use within a body vessel and methods of manufacturing. The medical catheter comprises a tubular catheter shaft having a distal end that fits within the body vessel. The tubular catheter shaft comprises an unfilled or low-loaded inner liner and/or outer shell. The medical catheter also comprises a radiopaque segment that comprises a radiopaque material embedded between the inner liner and outer shell. The radiopaque material can be in the form of an ink, powder, or paste.

US 6 213 995 B 1 describes a flexible tubing that includes a wall provided with a plurality of braided elements forming a braid within the wall of the tube. The braided elements include one or more signal transmitting elements and one or more metallic or non-metallic structural elements having structural properties different from the signal transmitting elements. The signal transmitting elements may be conductive wires, e.g., a sensor conductor or thermocouple, or optical fibers. Additionally, a combination of conductive wires and optical fibers may be provided within the braid. The structural elements preferably provide a degree of torsional stiffness, kink resistance, or luminal rigidity to the catheter which is different than would otherwise be provided solely with one or more signal transmitting elements. The tubing wall is preferably a cross-linking polymer, such as polyimide matrix. In manufacture of the tubing, one or more layers of the polymer are coated on a mandrel and permitted to cure to form an inner layer of the tubing. The braid is then woven about the inner layer. Then, one or more layers of the polymer are coated over the braid and inner layer and permitted to cure to form an outer layer of the tubing which preferably forms a cross-linked and adhesive bond to the inner layer and provides a relatively homogenous structure.

EP 0 277 366 A1 describes a guiding catheter assembly adapted to be inserted into a vessel having a first guiding catheter and a second guiding catheter removably disposed within the first guiding catheter. The second guiding catheter has a length so that it extends beyond the distal extremity of the first guiding catheter. Each of the first and second guiding catheters includes a flexible elongate tubular member having a bore extending therethrough and a fitting secured to the proximal extremity of the tubular member. The bore in the tubular member is of a size so that the portion of the second guiding catheter extending beyond the fitting can be introduced into the bore of the tubular member of the first guiding catheter.

US 5 782 760 A describes an over-the-wire electrophysiology catheter which has an emitting electrode on the distal tip electrically connected to a source of high frequency electrical energy. The intravascular device is configured to be advanced through a patient's cardiac veins or coronary arteries and preferably is also provided with sensing electrodes for detecting electrical activity of the patient's heart from within a blood vessel of the heart. The device forms large lesions in tissue adjacent to the blood vessel in which the device is located without significantly damaging the blood vessel to effectively terminate signals causing arrhythmia.

US 2010/268173 A1 describes a catheter, such as an intravascular catheter, that includes a longitudinally pre-oriented tubular substrate and reinforcing means surrounding and in contact with the substrate, the reinforcing means wound over the substrate, and a tubular superstrate surrounding the reinforcing means forcing the reinforcing means against the substrate and maintaining the reinforcing means wound over the substrate. The reinforcing means is a micro-tape having a width less than 0.5 mm and includes a filament layer having a cross sectional aspect ratio (width/height) of 2 to 20, wherein the filaments are fixated by a cured or solidified resin or wax, which constitutes 2 to 40 wt % of the micro-tape.

US 2005/137519 A1 describes composite catheter braids that can be formed from a plurality of filaments, some of which can include significant amounts of molybdenum. Filaments including stainless steel and other materials can be used in combination with filaments including molybdenum. Individual filaments can be composed of single metals, or can represent alloys. Composite catheter braids can be employed in intravascular catheters.

US 2012/277729 A1 describes a catheter having a selectively variable degree of flexibility includes an elongate tubular outer member and an elongate tubular inner member disposed within the outer member. The catheter further includes a cavity disposed in between the outer and inner members. A tubular braid of filaments may be disposed within the cavity. The outer and inner members are movable relative to each other along intermediate portions thereof when in a first configuration, and are not movable relative to each other when in a second configuration. When the inner and outer members are in the first configuration, the tubular braid of filaments is movably disposed relative to at least one of the outer member and the inner member. When the inner and outer members are in the second configuration, the inner and outer members clamp the tubular braid of filaments in a fixed position relative to the inner and outer members.

US 2013/046298 A1 describes apparatus that are provided for performing a procedure within a patient's body that include a tubular member including proximal and distal ends, a central axis or region extending therebetween, and a distal portion extending distally from an intermediate portion to the distal end. One or more accessory lumens and a steering element lumen extend between the proximal and distal ends, the steering element lumen offset from the central region within the distal portion such that a steering plane intersects the steering element lumen and the central axis. The distal portion includes a core member extending between the intermediate portion and the distal end, and stabilization elements embedded in the core member adjacent the accessory lumen and defining a stabilization plane that intersects the steering plane at a location offset from the central region, e.g., closer to the accessory lumen than the steering element lumen.

New flexible sheaths capable of withstanding high temperatures associated with ablation energy (e.g., microwave ablation energy) are needed.

### SUMMARY

The present invention relates to flexible sheathes configured to withstand high amounts of temperature during endoscopic energy delivery procedures, and related systems.

According to an aspect, there is provided a flexible sheath according to claim 1.

According to another aspect, there is provided a system according to claim 8.

Further features according to embodiments are defined in the dependent claims.

Embodiments of the invention can provide a sheath assembly having a flexible elongate tubular body designed with a temperature resistant polymer and/or a temperature resistant braided material. Such sheath assemblies are configured for use in any kind of endoscopic energy delivery procedure (e.g., tissue ablation, resection, cautery, vascular thrombosis, treatment of cardiac arrhythmias and dysrhythmias, electrosurgery, tissue harvest, etc.).

In some embodiments, the flexible sheath is able to withstand high amounts of temperature (e.g., from approximately 50 C to 150 C) during endoscopic energy delivery procedures without sustaining damage (e.g., structural damage). The flexile sheaths are designed to be operational within a microwave field or microwave zone (e.g., the flexible sheaths are microwave compatible) without sustaining microwave field or microwave zone related damage. The flexile sheaths are designed to be operational within a tissue region experiencing high temperatures (e.g., the flexible sheaths are thermal resistant) without sustaining high temperature related damage.

In some embodiments, the heat-resistant synthetic fiber is polyether ether ketone (PEEK), Kevlar, Aramid fibers, Nomex, and/or Technora.

In some embodiments, the braided body portion extends along the exterior of the elongate tubular body exterior, within the elongate tubular body, along the interior of the elongate tubular body, or a mixture of the interior and exterior of the elongate tubular body.

In some embodiments, the flexible sheath has sufficient flexibility to access a circuitous route through a subject (e.g., through a branched structure, through the bronchial tree, through any region of the body to reach a desired location) while retaining the ability to withstand high amounts of temperature during endoscopic energy delivery procedures without sustaining damage (e.g., structural damage).

In some embodiments, the composition of the elongate tubular body is a higher temperature rated polymer material. In some embodiments, the composition of the elongate tubular body is fluorinated ethylene propylene (FEP). In some embodiments, the higher temperature rated polymer material is a thermoplastic copolyester. In some embodiments, the thermoplastic copolyester is Arnitel. In some embodiments, the composition of the elongate tubular body is a fluoropolymer. In some embodiments, the fluoropolymer is perfluoromethylalkoxy alkane (MFA) or perfluoroalkoxy alkane (PFA).

In some embodiments, the flexible sheaths further comprise a marker region positioned at the elongate tubular body distal end. In some embodiments, the composition of the marker region is a high density ceramic. In some embodiments, the composition of the marker region is a high temperature resistant plastic substrate. In some embodiments, the composition of the marker region is a high temperature resistant plastic substrate with a high density ceramic coating.

In some embodiments, the flexible sheaths further comprise a steerable pull ring configured to permit a user to steer the flexible sheath in any desired manner.

In certain embodiments, the present invention provides systems comprising a primary catheter, a flexible sheath as described herein, and an energy delivery device. In some embodiments, the primary catheter is an endoscope. In some embodiments, the energy delivery device is a microwave energy delivery device.

Methods of treating a tissue region are disclosed, comprising providing a system comprising a primary catheter, a flexible sheath as described herein, and an energy delivery device, inserting the primary catheter into a tissue region, inserting the flexible sheath through the primary catheter to a desired tissue region to be treated, inserting the energy delivery device through the flexible sheath to the desired tissue region to be treated, and treating the tissue region to be treated with the energy delivery device. In some examples, the tissue region to be treated is within a subject. In some examples, the subject is a human subj ect.

Additional embodiments are described herein.

### BRIEF DESCRIPTON OF THE DRAWINGS

Fig. 1 shows how a standard flexible sheath is separated from an ablation energy device (probe)) for purposes avoiding exposure to high temperatures during a therapeutic endoscopy procedure.
Figs. 2-6 show various flexible sheath embodiments.

### DETAILED DESCRIPTION

Therapeutic endoscopy or interventional endoscopy pertains to an endoscopic procedure during which a treatment (e.g., tissue ablation) (e.g., tissue collection) is carried out via the endoscope. This contrasts with diagnostic endoscopy, where the aim of the procedure is purely to visualize an internal part of a body (e.g., gastrointestinal region, respiratory region, urinary tract region, etc.) in order to aid diagnosis. In practice, a procedure which starts as a diagnostic endoscopy may become a therapeutic endoscopy depending on the findings.

Generally, therapeutic endoscopy involves the administration of an endoscope ("primary catheter") into a body region until a natural stopping positioning is reached (e.g., until the circumference of the body region inhibits further advancement of the endoscope). Next, a flexible sheath having a circumference smaller than the circumference of the endoscope is advanced through the endoscope and to a desired body region location. Next, a therapeutic tool (e.g., an ablation energy delivery tool) (e.g., a tissue collection tool) having a circumference smaller than the diameter of the flexible sheath is advanced through the flexible sheath to the desired body region location. Next, in ablation energy delivery procedures, the flexible sheath is withdrawn so as to avoid potential high temperature exposure from the ablation energy delivery tool. Next, ablation energy is delivered to the desired body region location. Upon completion of the therapeutic endoscopy, the ablation energy delivery tool is withdrawn through the flexible sheath, the flexible sheath is withdrawn through the endoscope, and the endoscope is withdrawn from the subject.

Current or typical flexible sheaths are constructed with a polymer material (e.g., polyether block amide (Pebax)) and a metal braiding (e.g., a stainless steel metal braiding). Such a polymer material as Pebax is used due to its biocompatibility and flexibility. In addition, such a polymer material as Pebax is used due to its lower melting temperature which is beneficial during manufacturing. Such a metal braiding as a stainless steel metal braiding provides the flexible sheath with torsional stiffness and permits a user (e.g., a clinician) to freely turn and rotate the flexible sheath without it collapsing upon itself.

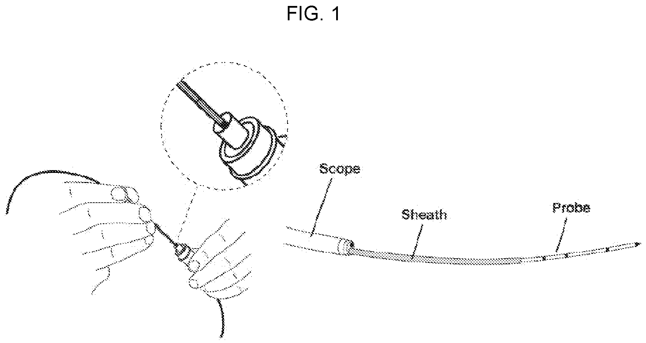

As noted, such flexible sheaths are used in therapeutic endoscopy procedures (e.g., endoscopic ablation procedures) to provide more precise access to a target location than an endoscope can provide. Current or typical flexible sheaths (e.g., having a polymer material (e.g., polyether block amide (Pebax)) and a metal braiding (e.g., a stainless steel metal braiding)) must be retracted in such endoscopic ablation procedures as such sheaths are incapable of handling high temperatures associated with ablation energy (e.g., microwave ablation energy). Indeed, such sheaths must be retracted prior to delivery of ablation energy to prevent damage to the sheath from the energy delivery (e.g., microwave field resulting from microwave energy delivery). Fig. 1 shows how such a standard sheath is separated from an ablation energy device (probe)) for purposes avoiding exposure to high temperatures. Such necessary flexible sheath retraction, however, often compromises the endoscopic ablation procedure through, for example, misadjusting the target location and/or misadjusting the positioning of the energy delivery device.

New flexible sheaths capable of withstanding high temperatures associated with ablation energy (e.g., microwave ablation energy) are needed.

The present invention addresses this need through providing flexible sheaths able to withstand high amounts of temperature (e.g., from approximately 50 C to 150 C) during endoscopic energy delivery procedures without sustaining damage (e.g., structural damage). The flexile sheaths are designed to be operational within a microwave field or microwave zone (e.g., the flexible sheaths are microwave compatible) without sustaining microwave field or microwave zone related damage. The flexile sheaths are designed to be operational within a tissue region experiencing high temperatures (e.g., the flexible sheaths are thermal resistant) without sustaining high temperature related damage.

Indeed, the present invention relates to flexible sheath assemblies configured to withstand high amounts of temperature during endoscopic energy delivery procedures, and related systems and methods of use. In particular, the present invention provides a sheath assembly having a flexible elongate tubular body designed with a temperature resistant polymer and/or a temperature resistant braided material. Such sheath assemblies are configured for use in any kind of endoscopic energy delivery procedure (e.g., tissue ablation, resection, cautery, vascular thrombosis, treatment of cardiac arrhythmias and dysrhythmias, electrosurgery, tissue harvest, etc.).

Accordingly, provided herein are flexible sheaths designed to withstand high amounts of temperature during endoscopic energy delivery procedures.

The flexible sheaths of the present invention are not limited to particular size dimensions. Indeed, in some embodiments, the size dimension of the flexible sheath is such that it is able to fit within and pass through the lumen of a primary catheter (e.g., an endoscope). In some embodiments, the flexible sheath is of sufficient diameter (e.g. 1 mm... 2 mm...3 mm...4 mm... 5 mm) to accommodate within and through its interior one or more suitable tools (e.g., energy delivery device, steerable navigation catheter). In some embodiments, the flexible sheath is of sufficient length to extend from an insertion site (e.g. mouth, incision into body of subject, etc.) to a desired target region within a living body (e.g. 50 cm...75 cm... 1 m... 1.5 m...2m...10m...25m, etc.). In some embodiments, the flexible sheath is of sufficient length to extend through and beyond the reach of a primary catheter (e.g., endoscope) to reach a treatment site (e.g. peripheral lung tissue, heart tissue, gastrointestinal tissue, etc.) (e.g., any desired location within a living body). In some embodiments, the size dimension of the flexible sheath is such that it is able to fit within and pass through the lumen of a primary catheter (e.g., an endoscope) while retaining the ability to withstand high amounts of temperature during endoscopic energy delivery procedures without sustaining damage (e.g., structural damage).

The flexible sheaths of the present invention are not limited to a particular manner of navigation through a primary catheter and/or through a body region. In some embodiments, the flexible sheath comprises a navigation and/or steering mechanism. In some embodiments, the flexible sheath is without an independent means of navigation, position recognition, or maneuvering. In some embodiments, the flexible sheath relies upon the primary catheter (e.g., endoscope) or a steerable navigation catheter for placement.

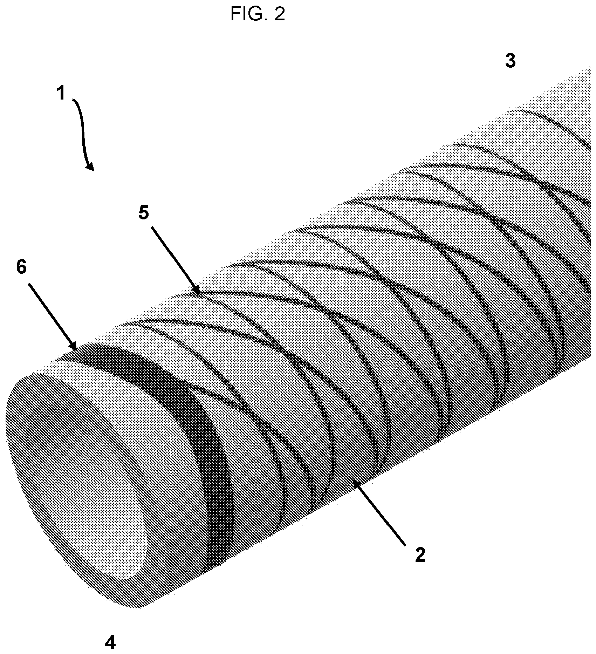

Fig. 2 shows a flexible sheath **1** embodiment of the present invention. The flexible sheath **1** is not limited to a particular design or configuration. In some embodiments, the design or configuration of the flexible sheath **1** is such that it is able to withstand high amounts of temperature during endoscopic energy delivery procedures without sustaining damage (e.g., structural damage).

In certain embodiments, as shown in Fig. 2, the flexible sheath **1** has an elongate tubular body **2** having an elongate tubular body proximal end **3** and an elongate tubular body distal end **4,** a braided body portion **5,** and (optionally) a marker region **6.** Such embodiments are not limited to a particular positioning for the elongate tubular body **2,** the braided body portion **5,** and (optionally) the marker region **6** within the flexible sheath **1.** In some embodiments, the elongate tubular body **2,** the braided body portion **5,** and (optionally) the marker region **6** are positioned within the flexible sheath **1** in any manner which permits the resulting embodiment able to withstand high amounts of temperature during endoscopic energy delivery procedures without sustaining damage (e.g., structural damage).

Still referring to Fig. 2, the elongate tubular body **2** is not limited to a particular composition. In some embodiments, the composition of the elongate tubular body **2** is any composition that renders the flexible sheath **1** able to withstand high amounts of temperature during endoscopic energy delivery procedures without sustaining damage (e.g., structural damage). In some embodiments, the composition of the elongate tubular body **2** is a higher temperature rated polymer material. Such embodiments are not limited to a particular higher temperature rated polymer material. In some embodiments, the higher temperature rated polymer material is fluorinated ethylene propylene (FEP). In some embodiments, the higher temperature rated polymer material is a thermoplastic copolyester. In some embodiments, the thermoplastic copolyester is Arnitel. In some embodiments, the higher temperature rated polymer material is a fluoropolymer. Such embodiments are not limited to a particular fluoropolymer. In some embodiments, the fluoropolymer is perfluoromethylalkoxy alkane (MFA). In some embodiments, the fluoropolymer is perfluoroalkoxy alkane (PFA). In some embodiments, only a portion (5%, 10%, 25%, 50%, 75%, 77%, 79%, 85%, 88%, 90%, 94%, 98%, 99%, 99.999%) of the elongate tubular body **2** has a composition of a higher temperature rated polymer material. In some embodiments, only a portion (5%, 10%, 25%, 50%, 75%, 77%, 79%, 85%, 88%, 90%, 94%, 98%, 99%, 99.999%) starting from the elongate tubular body distal end **4** has a composition of a higher temperature rated polymer material. In some embodiments, the entire elongate tubular body **2** has a composition of a higher temperature rated polymer material.

Still referring to Fig. 2, the elongate tubular body **2** has an elongate tubular body proximal end **3** positioned at its proximal end, and an elongate tubular body distal end **4** positioned at its distal end. As can be seen, both the elongate tubular body proximal end **3** and elongate tubular body distal end **4** serve as openings for the elongate tubular body **2** and the flexible sheath **1** which represents a natural openings through which suitable tools (e.g., energy delivery device, steerable navigation catheter) can be accommodated into the flexible sheath **1,** passed through the flexible sheath **1,** and positioned outside of the flexible sheath **1.**

Still referring to Fig. 2, the braided body portion **5** is not limited to a particular composition. As noted, current designs of flexible sheaths utilize a metal based braid to provide flexibility to the sheath. Such metal based braids, however, when exposed to high energy fields (e.g., microwave fields) elevate in temperature rapidly thereby damaging the sheath composition (e.g., melting the sheath). As such, the composition of the braided body portion **5** for the flexible sheaths **1** of the present invention is any composition that renders the flexible sheath **1** able to withstand high amounts of temperature during endoscopic energy delivery procedures without sustaining damage (e.g., structural damage). In some embodiments, the composition of the braided body portion **5** is a heat-resistant synthetic fiber. Such embodiments are not limited to a particular heat-resistant synthetic fiber. In some embodiments, the heat-resistant synthetic fiber is polyether ether ketone (PEEK), Kevlar, Aramid fibers, Nomex, and/or Technora.

Still referring to Fig. 2, the braided body portion **5** is not limited to a particular positioning within the flexible sheath **1.** In some embodiments, the braided body portion **5** is positioned along the length of the elongate tubular body **2** from the elongate tubular body proximal end **3** to the elongate tubular body distal end **4.** In some embodiments, the braided body portion **5** is positioned within the interior of the elongate tubular body **2** (such that it is not visible from the exterior or interior of the flexible sheath **1**). In some embodiments, the braided body portion **5** is positioned along the exterior of the elongate tubular body **2** (such that it is visible from the exterior but not interior of the flexible sheath **1**). In some embodiments, the braided body portion **5** is positioned within and along the exterior of the elongate tubular body **2** (such that it is visible from the exterior but not interior of the flexible sheath **1**). In some embodiments, the braided body portion **5** is positioned along the interior of the elongate tubular body **2** (such that it is visible from the interior but not exterior of the flexible sheath **1**). In some embodiments, the braided body portion **5** is positioned within and along the interior of the elongate tubular body **2** (such that it is visible from the interior but not exterior of the flexible sheath **1**). In some embodiments, the braided body portion **5** is positioned within and along the interior and exterior of the elongate tubular body **2** (such that it is visible from the interior and exterior of the flexible sheath **1**).

Still referring to Fig. 2, the braided body portion **5** is not limited to a particular braid design. In some embodiments, the design of the braided body portion **5** is able to withstand high amounts of temperature during endoscopic energy delivery procedures without sustaining damage (e.g., structural damage).

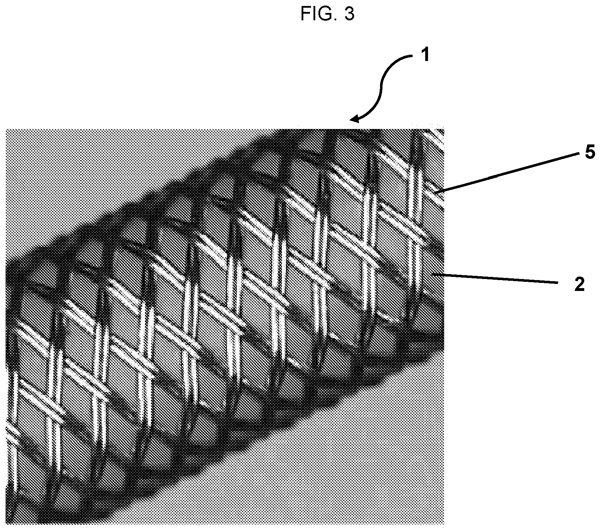

Fig. 3 shows a standard braid design for braided body portion **5** of a flexible sheath **1.** As can be seen, the braided body portion **5** is positioned within and along the interior of the elongate tubular body **2.**

Referring again to Fig. 2, the flexible sheaths **1** are not limited to having a particular amount of flexibility provided by the braided body portion **5.** In some embodiments, the flexible sheaths **1** have sufficient flexibility to access a circuitous route through a subject (e.g., through a branched structure, through the bronchial tree, through any region of the body to reach a desired location) while retaining the ability to withstand high amounts of temperature during endoscopic energy delivery procedures without sustaining damage (e.g., structural damage).

Referring again to Fig. 2, the flexible sheaths **1** have therein a marker region **6.** It is common for flexible sheaths used in endoscopy procedures to have a "marker" at its distal end which provides a user (e.g., a clinician) the ability to visualize the distal end of the sheath during a procedure (e.g., via any imaging technique) (e.g., via x-ray). Generally, such sheaths utilize a dense material radiopaque material for such a maker. As with polymer compositions and braided portions that are unable to withstand high temperatures, dense material radiopaque materials are susceptible to elevating in temperature when exposed to energy ablation fields (e.g., microwave ablation fields). As shown in Fig. 1, the flexible sheaths **1** of the present invention overcome this limitation through providing a marker region **6** able to withstand high amounts of temperature during endoscopic energy delivery procedures without sustaining damage (e.g., structural damage).

Still referring to Fig. 2, the marker region **6** is not limited to a particular composition. In some embodiments, the composition of the marker region **6** is such that it renders the region able to withstand high amounts of temperature during endoscopic energy delivery procedures without sustaining damage (e.g., structural damage). In some embodiments, the composition of the marker region **6** is ceramic. Such embodiments are not limited to a particular type of ceramic. In some embodiments, the ceramic is a high density ceramic. In some embodiments, the marker region **6** is a high temperature resistant plastic substrate. In some embodiments, the marker region **6** is a high temperature resistant plastic substrate having a ceramic coating (e.g., a high density ceramic coating).

Still referring to Fig. 2, the marker region **6** is not limited to a particular position within the flexible sheath **1.** In some embodiments, the maker region **6** is positioned at the elongate tubular body distal end **4.** In some embodiments, the marker region **6** extends along the entire outside circumference of the elongate tubular body distal end **4.** In some embodiments, the marker region **6** extends along only a portion (e.g., 1%, 2%, 5%, 25%, 50%, 75%, 77%, 78%, 82%, 95%, 99%, 99.9999%) the entire outside circumference of the elongate tubular body distal end **4.** In some embodiments, the marker region **6** is positioned along the exterior of the elongate tubular body distal end **6** such that it is visible from exterior but not the interior of the flexible sheath **1.** In some embodiments, the marker region **6** is positioned along the interior of the elongate tubular body distal end **6** such that it is visible from interior but not the exterior of the flexible sheath **1.** In some embodiments, the marker region **6** is positioned within the elongate tubular body distal end **6** such that it not visible from interior or the exterior of the flexible sheath **1.**

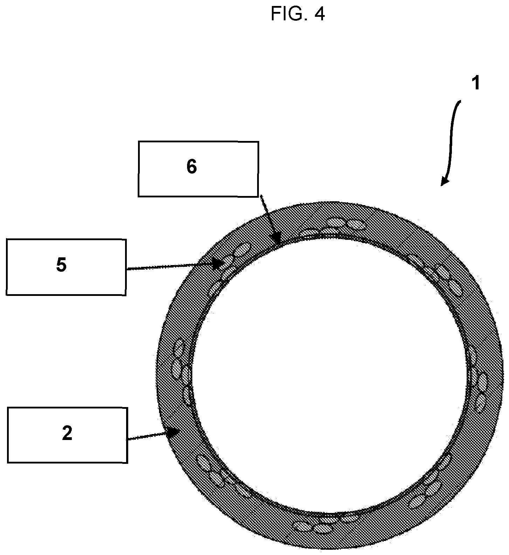

Referring to Fig. 4, a flexible sheath **1** embodiment is shown looking through the elongate tubular body distal end **4** of the elongate tubular body **2.** As shown, the maker region **6** is positioned along the interior of the elongate tubular body distal end **4.** As shown the braided body portion **5** is positioned within the elongate tubular body **2.**

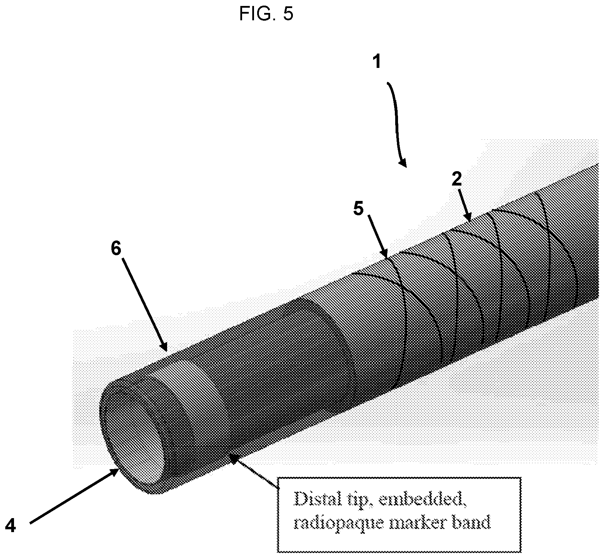

Referring to Fig. 5, a flexible sheath **1** embodiment is shown from a top down angled perspective. As can be seen, a marker region **6** is positioned along the exterior of the elongate tubular body distal end **4.** As can be seen, the braided body portion **5** is positioned along the exterior of the elongate tubular body **2.**

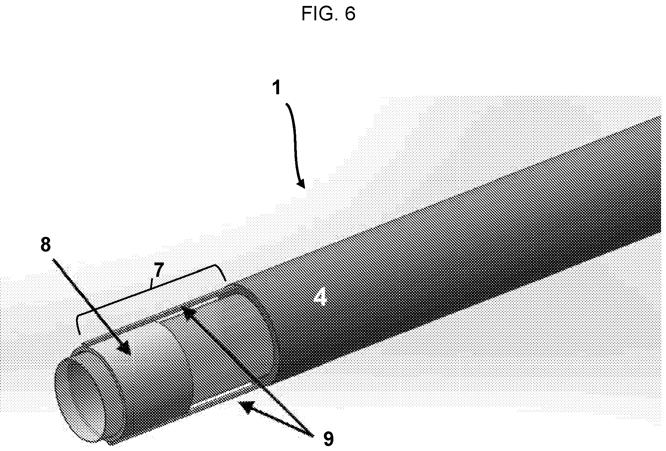

Referring to Fig. 6, in some embodiments, the flexible sheaths **1** further contain a steerable pull ring **7.** As shown in Fig. 6, a flexible sheath **1** is shown with a steerable pull ring **7** positioned at the elongate tubular body distal end **4.** Such embodiments are not limited to a particular configuration for the steerable pull ring **7.** In some embodiments, the steerable pull ring **7** has any configuration that permits a user to manually steer the flexible sheath **1** via manipulation of the steerable pull ring **7** (e.g., manipulation of one or both of the wires results in a curving or steering of the sheath).

In some embodiments, the steerable pull ring **7** permits the flexible sheath **1** to be steered in any desired manner or direction. For example, in some embodiments, the steerable pull ring **7** permits the flexible sheath **1** to be steered at any desired curve angle (e.g., from 1 to 180 degrees). In some embodiments, the steerable pull ring **7** permits the flexible sheath **1** to be steered at any desired bend angle (e.g., from 1 to 360 degrees). In some embodiments, the steerable pull ring **7** permits the flexible sheath **1** to be steered at any desired bend radius (e.g., from 1 to 360 degrees). In some embodiments, the steerable pull ring **7** permits the flexible sheath **1** to be steered at any desired curve diameter. In some embodiments, the steerable pull ring **7** permits the flexible sheath **1** to be steered at any desired reach (e.g., from .1 to 100 mm). In some embodiments, the steerable pull ring **7** permits the flexible sheath **1** to be steered at any desired curl. In some embodiments, the steerable pull ring **7** permits the flexible sheath **1** to be steered at any desired sweep. In some embodiments, the steerable pull ring **7** permits the flexible sheath **1** to be steered at any desired curve (e.g., symmetrical or asymmetrical) (e.g., multi-curve or compound curve). In some embodiments, the steerable pull ring **7** permits the flexible sheath **1** to be steered at any desired loop. In some embodiments, the steerable pull ring **7** permits the flexible sheath **1** to be steered at any desired deflection (e.g., on-plane deflection, off plane deflection).

Still referring to Fig. 6, the steerable pull ring **7** has a ring portion **8** and two wires **9.** As shown, the ring portion **8** completely wraps around the elongate tubular body distal end **4** and serves as a leverage point for the two wires **9.** The two wires **9** are positioned to extend along the entire length of the flexible sheath **1** from the elongate tubular body distal end **4** to the elongate tubular body proximal end **4** where the two wires **9** can be manipulated by a user which thereby results in a steering of the flexible sheath **1.** In some embodiments, the ring portion **8** is resistant to high temperatures (e.g., temperatures resulting from ablation energy). In some embodiments, the composition of the ring portion **8** is polyether ether ketone (PEEK), Kevlar, Aramid fibers, Nomex, and/or Technora. In some embodiments, the composition of the ring portion **8** is ceramic. In some embodiments, the composition of the ring portion **8** is non-metallic. In some embodiments, the two wires are attached to the ring portion **8** such that pulling of one or both of the two wires **9** will not detach the two wires **9** from the ring portion **8.** In some embodiments, the two wires **9** are attached to the exterior of the ring portion **9.** In some embodiments, the two wires **9** are attached to the interior of the ring portion **9.** In some embodiments, shown in Fig. 6, the two wires **9** are positioned in an opposed manner (e.g., directly across from each other). In some embodiments, more than two wires are provided (e.g., 3, 4, 5, 6, 10, 15, 20, etc). In some embodiments, only one wire is provided (e.g., 3, 4, 5, 6, 10, 15, 20, etc). In some embodiments, the two wires **9** are resistant to high temperatures (e.g., temperatures resulting from ablation energy). In some embodiments, the composition of the two wires **9** is polyether ether ketone (PEEK), Kevlar, Aramid fibers, Nomex, and/or Technora. In some embodiments, the composition of the two wires **9** is ceramic. In some embodiments, the composition of the two wires **9** is non-metallic.

In some embodiments, the present invention provides systems for therapeutic endoscopic procedures wherein flexible sheaths as described herein, primary catheters, and one or more suitable tools (e.g., energy delivery device, steerable navigation catheter) are provided.

Such embodiments are not limited to a particular type or kind of primary catheter. In some embodiments, the present invention primary catheter is an endoscope. In some embodiments, any suitable endoscope known to those in the art finds use as a primary catheter in the present invention. In some embodiments, a primary catheter adopts characteristics of one or more endoscopes and/or bronchoscopes known in the art, as well as characteristics described herein. One type of conventional flexible bronchoscope is described in U.S. Pat. No. 4,880,015. The bronchoscope measures 790 mm in length and has two main parts, a working head and an insertion tube. The working head contains an eyepiece; an ocular lens with a diopter adjusting ring; attachments for suction tubing, a suction valve, and light source; and an access port or biopsy inlet, through which various devices and fluids can be passed into the working channel and out the distal end of the bronchoscope. The working head is attached to the insertion tube, which typically measures 580 mm in length and 6.3 mm in diameter. The insertion tube contains fiberoptic bundles, which terminate in the objective lens at the distal tip, light guides, and a working channel. Other endoscopes and bronchoscopes which may find use in embodiments of the present invention, or portions of which may find use with the present invention, are described in U.S. Pat. No. 7,473,219; U.S. Pat. No. 6,086,529; U.S. Pat. No. 4,586,491; U.S. Pat. No. 7,263,997; U.S. Pat. No. 7,233,820; and U.S. Pat. No. 6,174,307.

Such embodiments are not limited to a particular type or kind of steerable navigation catheter. In some embodiments, a steerable navigation catheter is configured to fit within the lumen of a primary catheter (e.g., endoscope) and a flexible sheath. In some embodiments, a steerable navigation catheter is of sufficient length to extend from an insertion site (e.g. mouth, incision into body of subject, etc.) to a treatment site (e.g. 50 cm...75 cm... 1 m... 1.5 m...2m... 5m... 15m). In some embodiments, a channel catheter is of sufficient length to extend beyond the reach of a primary catheter (e.g., endoscope) to reach a treatment site (e.g. peripheral lung tissue). In some embodiments, a steerable navigation catheter engages a flexible sheath such that movement of the steerable navigation catheter results in synchronous movement of the flexible sheath. In some embodiments, as a steerable navigation catheter is inserted along a path in a subject, the flexible sheath surrounding the steerable navigation catheter moves with it. In some embodiments, a flexible sheath is placed within a subject by a steerable navigation catheter. In some embodiments, a steerable navigation catheter can be disengaged from a flexible sheath. In some embodiments, disengagement of a steerable navigation catheter and flexible sheath allows movement of the steerable navigation catheter further along a pathway without movement of the flexible sheath. In some embodiments, disengagement of a steerable navigation catheter and flexible sheath allows retraction of the steerable navigation catheter through the flexible sheath without movement of the flexible sheath.

Such embodiments are not limited to a particular type or kind of energy delivery device (e.g., ablation device, surgical device, etc.) (see, e.g., U.S. Patent Nos. 7,101,369, 7,033,352, 6,893,436, 6,878,147, 6,823,218, 6,817,999, 6,635,055, 6,471,696, 6,383,182, 6,312,427, 6,287,302, 6,277,113, 6,251,128, 6,245,062, 6,026,331, 6,016,811, 5,810,803, 5,800,494, 5,788,692, 5,405,346, 4,494,539, U.S. Patent Application Serial Nos. 11/728,460, 11/728,457, 11/728,428, 11/237,136, 11/236,985, 10/980,699, 10/961,994, 10/961,761, 10/834,802, 10/370,179, 09/847,181; Great Britain Patent Application Nos. 2,406,521, 2,388,039; European Patent No. 1395190; and International Patent Application Nos. WO 06/008481, WO 06/002943, WO 05/034783, WO 04/112628, WO 04/033039, WO 04/026122, WO 03/088858, WO 03/039385 WO 95/04385). Such energy delivery devices are not limited to emitting a particular kind of energy. In some embodiments, the energy delivery devices are capable of emitting radiofrequency energy. In some embodiments, the energy delivery devices are capable of emitting microwave energy. Such devices include any and all medical, veterinary, and research applications devices configured for energy emission, as well as devices used in agricultural settings, manufacturing settings, mechanical settings, or any other application where energy is to be delivered.

The systems for therapeutic endoscopic procedures of the present invention are not limited to particular uses. Indeed, such systems of the present invention are designed for use in any setting wherein the emission of energy is applicable. Such uses include any and all medical, veterinary, and research applications. In addition, the systems and devices of the present invention may be used in agricultural settings, manufacturing settings, mechanical settings, or any other application where energy is to be delivered.

In some embodiments, the systems are configured any type of procedure wherein the flexible sheath described herein can find use. For example, the systems find use for open surgery, percutaneous, intravascular, intracardiac, intraluminal, endoscopic, laparoscopic, or surgical delivery of energy.

The present invention is not limited by the nature of the target tissue or region. Uses include, but are not limited to, treatment of heart arrhythmia, tumor ablation (benign and malignant), control of bleeding during surgery, after trauma, for any other control of bleeding, removal of soft tissue, tissue resection and harvest, treatment of varicose veins, intraluminal tissue ablation (e.g., to treat esophageal pathologies such as Barrett's Esophagus and esophageal adenocarcinoma), treatment of bony tumors, normal bone, and benign bony conditions, intraocular uses, uses in cosmetic surgery, treatment of pathologies of the central nervous system including brain tumors and electrical disturbances, sterilization procedures (e.g., ablation of the fallopian tubes) and cauterization of blood vessels or tissue for any purposes. In some embodiments, the surgical application comprises ablation therapy (e.g., to achieve coagulative necrosis). In some embodiments, the surgical application comprises tumor ablation to target, for example, metastatic tumors. In some embodiments, the systems including the flexible sheath described herein are configured for movement and positioning, with minimal damage to the tissue or organism, at any desired location, including but not limited to, the lungs, brain, neck, chest, abdomen, and pelvis. In some embodiments, the systems are configured for guided delivery, for example, by computerized tomography, ultrasound, magnetic resonance imaging, fluoroscopy, and the like. Indeed, in some embodiments, all inserted components of such a system are configured for movement along a narrow and circuitous path through a subject (e.g. through a branched structure, through the bronchial tree, etc.).

Methods of treating a tissue region are disclosed, comprising providing a tissue region and a system described herein (e.g., a primary catheter (e.g., an endoscope), a flexible sheath as described herein, and an energy delivery device (e.g., a microwave ablation catheter), and at least one of the following components: a processor, a power supply, a temperature monitor, an imager, a tuning system, a temperature reduction system, and/or a device placement system); positioning a portion of the energy delivery device in the vicinity of the tissue region, and delivering an amount of energy with the device to the tissue region. In some examples, the tissue region is a tumor. In some examples, the delivering of the energy results in, for example, the ablation of the tissue region and/or thrombosis of a blood vessel, and/or electroporation of a tissue region. In some examples, the tissue region is a tumor. In some examples, the tissue region comprises one or more of the lung, heart, liver, genitalia, stomach, lung, large intestine, small intestine, brain, neck, bone, kidney, muscle, tendon, blood vessel, prostate, bladder, and spinal cord.

## Claims

1. A flexible sheath for use in endoscopic procedures, wherein the flexible sheath comprises:
an elongate tubular body (2) having an elongate tubular body proximal end and an elongate tubular body distal end;
a braided body portion (5) extending from the elongate tubular body distal end to the elongate tubular body proximal end,
a marker region (6) positioned at the elongate tubular body distal end,
wherein the composition of the braided body portion is a heat-resistant synthetic fiber,
wherein the diameter of the flexible sheath is less than 5 mm, and
wherein the composition of the marker region is a high temperature resistant plastic substrate with a high density ceramic coating.

2. The flexible sheath of claim 1, wherein the heat-resistant synthetic fiber comprises polyether ether ketone, Kevlar, Aramid fibers, Nomex, and/or Technora.

3. The flexible sheath of claim 1, wherein the braided body portion extends along the exterior of the elongate tubular body exterior, within the elongate tubular body, along the interior of the elongate tubular body, or a mixture of the interior and exterior of the elongate tubular body.

4. The flexible sheath of claim 1, wherein the composition of the elongate tubular body is fluorinated ethylene propylene or a thermoplastic copolyester, preferably Arnitel.

5. The flexible sheath of claim 1, wherein the composition of the elongate tubular body is a fluoropolymer.

6. The flexible sheath of claim 5, wherein the fluoropolymer is perfluoromethylalkoxy alkane or perfluoroalkoxy alkane

7. The flexible sheath of claim 1, further comprising a steerable pull ring configured to permit a user to steer the flexible sheath.

8. A system comprising a primary catheter, a flexible sheath as described in claim 1, and an energy delivery device.

9. The system of claim 8, wherein the primary catheter is an endoscope.

10. The system of claim 8, wherein the energy delivery device is a microwave energy delivery device.

## Patentansprüche

1. Eine flexible Hülse zur Verwendung in endoskopischen Prozeduren, wobei die flexible Hülse Folgendes beinhaltet:
einen länglichen röhrenförmigen Körper (2), der ein proximales Ende des länglichen röhrenförmigen Körpers und ein distales Ende des länglichen röhrenförmigen Körpers aufweist;
einen geflochtenen Körperabschnitt (5), der sich von dem distalen Ende des länglichen röhrenförmigen Körpers zu dem proximalen Ende des länglichen röhrenförmigen Körpers erstreckt,
einen Markierungsbereich (6), der an dem distalen Ende des länglichen röhrenförmigen Körpers positioniert ist,
wobei die Zusammensetzung des geflochtenen Körperabschnitts eine hitzebeständige synthetische Faser ist,
wobei der Durchmesser der flexiblen Hülse weniger als 5 mm beträgt und
wobei die Zusammensetzung des Markierungsbereichs ein hochtemperaturbeständiges Kunststoffsubstrat mit einer hochdichten Keramikbeschichtung ist.

2. Flexible Hülse gemäß Anspruch 1, wobei die hitzebeständige synthetische Faser Polyetheretherketon, Kevlar, Aramidfasern, Nomex und/oder Technora beinhaltet.

3. Flexible Hülse gemäß Anspruch 1, wobei sich der geflochtene Körperabschnitt entlang der Außenseite der Außenseite des länglichen röhrenförmigen Körpers, innerhalb des länglichen röhrenförmigen Körpers, entlang der Innenseite des länglichen röhrenförmigen Körpers oder einer Mischung aus der Innenseite und der Außenseite des länglichen röhrenförmigen Körpers erstreckt.

4. Flexible Hülse gemäß Anspruch 1, wobei die Zusammensetzung des länglichen röhrenförmigen Körpers fluoriertes Ethylenpropylen oder ein thermoplastischer Copolyester, vorzugsweise Arnitel, ist.

5. Flexible Hülse gemäß Anspruch 1, wobei die Zusammensetzung des länglichen röhrenförmigen Körpers ein Fluorpolymer ist.

6. Flexible Hülse gemäß Anspruch 5, wobei das Fluorpolymer ein Perfluormethylalkoxyalkan oder Perfluoralkoxyalkan ist.

7. Flexible Hülse gemäß Anspruch 1, die ferner einen lenkbaren Zugring beinhaltet, der konfiguriert ist, um einem Benutzer das Lenken der flexiblen Hülse zu ermöglichen.

8. Ein System, das einen Primärkatheter, eine flexible Hülse, wie in Anspruch 1 beschrieben, und eine Energiezuführungsvorrichtung beinhaltet.

9. System gemäß Anspruch 8, wobei der Primärkatheter ein Endoskop ist.

10. System gemäß Anspruch 8, wobei die Energiezuführungsvorrichtung eine Mikrowellenenergiezuführungsvorrichtung ist.

## Revendications

1. Une gaine souple destinée à être utilisée dans des procédures endoscopiques, la gaine souple comprenant :
un corps tubulaire allongé (2) ayant une extrémité proximale de corps tubulaire allongé et une extrémité distale de corps tubulaire allongé ;
une partie formant corps tressé (5) s'étendant de l'extrémité distale de corps tubulaire allongé à l'extrémité proximale de corps tubulaire allongé,
une région formant marqueur (6) située au niveau de l'extrémité distale de corps tubulaire allongé,
où la composition de la partie formant corps tressé est une fibre synthétique résistante à la chaleur,
où le diamètre de la gaine souple est de moins de 5 mm, et
où la composition de la région formant marqueur est un substrat plastique résistant aux températures élevées avec un revêtement céramique haute densité.

2. La gaine souple de la revendication 1, où la fibre synthétique résistante à la chaleur comprend du polyéther éther cétone, du Kevlar, des fibres aramides, du Nomex, et/ou du Technora.

3. Le gaine souple de la revendication 1, où la partie formant corps tressé s'étend sur l'extérieur de l'extérieur de corps tubulaire allongé, au sein du corps tubulaire allongé, sur l'intérieur du corps tubulaire allongé, ou un mélange de l'intérieur et de l'extérieur du corps tubulaire allongé.

4. La gaine souple de la revendication 1, où la composition du corps tubulaire allongé est de l'éthylène propylène fluoré ou un copolyester thermoplastique, préférablement de l'Arnitel.

5. La gaine souple de la revendication 1, où la composition du corps tubulaire allongé est un fluoropolymère.

6. La gaine souple de la revendication 5, où le fluoropolymère est le perfluorométhylalkoxy alcane ou le perfluoroalkoxy alcane.

7. La gaine souple de la revendication 1, comprenant en outre un anneau de traction orientable configuré pour permettre à un utilisateur d'orienter la gaine souple.

8. Un système comprenant un cathéter primaire, une gaine souple telle que décrite dans la revendication 1, et un dispositif d'administration d'énergie.

9. Le système de la revendication 8, où le cathéter primaire est un endoscope.

10. Le système de la revendication 8, où le dispositif d'administration d'énergie est un dispositif d'administration d'énergie micro-onde.
